# EUROPEAN PATENT APPLICATION

(11) **EP 4 209 135 A1**
(43) Date of publication of application: **12.07.2023**
(21) Application number: 22170604.7
(22) Date of filing: 28.04.2022
(51) Int. Cl.: A23L 33/12, A23L 33/15

(54) **COMPOSITION FOR REMOVING HANGOVER AND IMPROVING LIVER FUNCTION**

(30) Priority: 05.01.2022 KR 20220001658
(71) Applicant: Zefit Inc., Daegu 43017 (KR)
(72) Inventor: SHIN, Jun Nyeong, 38671 Gyeongsan-si (KR); LEE, Ki Baek, 42995 Daegu (KR)
(74) Representative: Vossius & Partner Patentanwälte Rechtsanwälte mbB

(57) **Abstract**

Provided is a composition for removing a hangover or improving liver function. The composition according to an embodiment can show an excellent hangover removal effect, and thus can be effectively used in a food composition or health functional food composition for removing or ameliorating a hangover and improving liver function.

## Description

### CROSS-REFERENCE TO RELATED APPLICATION

This application is based on and claims priority to Korean Patent Application No. 10-2022-0001658, filed on Jan 05, 2022, in the Korean Intellectual Property Office, the disclosure of which is incorporated by reference herein in its entirety.

### BACKGROUND

### 1. Field

The present disclosure relates to a composition for removing a hangover or improving liver function, and more particularly, to a food composition for removing or ameliorating a hangover, including the composition, a health functional food composition for removing or ameliorating a hangover, a food composition for improving liver function, and a health functional food for improving liver function.

### 2. Description of the Related Art

According to the OECD statistics on alcoholic beverage consumption per capita in 2019, Korea shows the highest alcohol consumption in Asia. In Korea, the monthly drinking rate for men over the age of 20 is 73.4%, and the monthly drinking rate for women is also 48.4%, which are generally high, and they are seriously exposed to excessive drinking due to the wrong drinking culture. However, excessive alcohol intake causes hangover symptoms, which are side effects of, for example, discomfort, headache, vomiting, decreased mental and physical work capacity, gastrointestinal pain, and dry mouth. This hangover phenomenon causes the side effects as alcohol (ethanol) is metabolized to acetaldehyde, known as a toxic substance, and accumulated in the body.

Ethanol introduced into the body is absorbed in the stomach or small intestine, moves through blood vessels to the liver, is oxidized to acetaldehyde by alcohol dehydrogenase (ADH), and is then decomposed to acetic acid in mitochondria by aldehyde dehydrogenase 2 (ALDH2). As such, when a large amount of ethanol is introduced into the body due to excessive drinking, non-oxidized ethanol and/or acetaldehyde is accumulated in the body and interferes with normal metabolism, causing various hangover symptoms.

Meanwhile, alcohol intolerance due to deficiency or mutation of aldehyde dehydrogenase, which breaks down acetaldehyde into acetic acid, is found only in Far East Asians, including Korea, China and Japan. According to the research results, variants of aldehyde dehydrogenase are found in about 35% of the Korean population.

As described above, Korea has the most developed hangover cure market in the world due to high alcohol consumption and genetic characteristics, and it was investigated that the domestic hangover cure market, which was around 130 billion won in 2015, had grown by more than 10% every year, reaching 250 billion won in 2019. Therefore, research and experiments are being conducted in various fields to alleviate hangovers caused by excessive drinking, and various products are actually being sold on the market.

Currently, the main ingredients used in hangover removing agents are plant extracts or medicinal herb extracts, including an oriental raisin tree fruit extract, a fermented rice germ extract, or the like, which are known to be effective in decomposing alcohol, taurine, vitamins, and the like. However, the amounts of the ingredients are small, and verification of the actual hangover removal efficacy of these compositions is also insufficient. Therefore, there is an urgent need to develop an active ingredient that can be verified to have significant hangover removal efficacy.

### SUMMARY

An aspect provides a food composition or health functional food composition for removing or ameliorating a hangover, including omega-3 fatty acid as an active ingredient.

In one embodiment, the food composition or the health functional food composition further includes vitamin B2 (riboflavin) and vitamin B3 (nicotinamide).

Another aspect provides a method of removing a hangover, including administering, to a subject in need thereof, a composition including an effective amount of omega-3 fatty acid.

Another aspect provides a food composition for use in removing a hangover, including omega-3 fatty acid as an active ingredient.

Another aspect provides a food composition or health functional food composition for improving liver function, including omega-3 fatty acid as an active ingredient.

In one embodiment, the food composition or the health function food composition further includes vitamin B2 (riboflavin) and vitamin B3 (nicotinamide).

Another aspect provides a pharmaceutical composition for improving liver function or preventing or treating liver damage, including omega-3 fatty acid as an active ingredient.

Additional aspects will be set forth in part in the description which follows and, in part, will be apparent from the description, or may be learned by practice of the presented embodiments of the disclosure.

Each description and embodiment provided in the present disclosure may also be applied to other descriptions and embodiments. That is, all combinations of various elements described in the present disclosure fall within the scope of the present disclosure. In addition, it cannot be considered that the scope of the present disclosure is limited by the specific descriptions described below.

Hereinafter, configurations of the present disclosure will be described in detail.

An aspect provides a food composition for removing or ameliorating a hangover, including omega-3 fatty acid as an active ingredient.

The omega-3 fatty acids are polyunsaturated fatty acids which include α-linolenic acid(ALA), eicosapentaenoic acid(EPA) or docosahexaenoic acid(DHA) and play a crucial role in human diet and physiology. For docosahexaenoic acid(DHA), as an example, it is primarily located in the cell membranes of the brain's retina, central nervous system tissue, heart muscle, or the like as a structural component and has a great effect on activating brain cells. According to the recent research results, it has been found that high intake of omega-3 fatty acids can treat or prevent stress disorder, bipolar depression, alcoholism, and the like.

The food composition for removing or ameliorating a hangover may further include vitamin B2 (riboflavin) and vitamin B3 (nicotinamide).

The food composition for removing or ameliorating a hangover may contain vitamin B2, vitamin B3, and omega-3 fatty acid in a concentration ratio of 1:0.5-9:5-14 with respect to the total concentration of the composition, but the present disclosure is not limited thereto.

As used herein, the terms "vitamin B2 (riboflavin)" and "vitamin B3 (nicotinamide)" act as coenzymes in various metabolic processes and play an important role in converting carbohydrates and fats into energy, and in particular, also act as coenzymes of alcohol dehydrogenase (ADH) or aldehyde dehydrogenase (ALDH) to accelerate the breakdown of alcohol. In addition, vitamin B2 and vitamin B3 have a protective and detoxifying action for liver cells, and may have a fatigue recovery action.

As described herein, the term "active ingredient" may refer to a bioactive material which is used for a substance mentioned herein (e.g., omega-3 fatty acid, vitamin B2, or vitamin B3), i.e., a bioactive substance to achieve functional efficacy mentioned herein (e.g., removing or prevention of hangover, and liver function improvement). This is distinguished from the treatment of omega-3 fatty acid, vitamin B2, or vitamin B3 alone, the treatment thereof in combination with other substances, or the additional treatment thereof, for the purpose of the known effect of omega-3 fatty acid, vitamin B2, or vitamin B3 (e.g., stimulation of alcohol metabolism, a protective and detoxifying action on liver cells, or fatigue recovery) to embody the effect mentioned herein. That is, the omega 3 fatty acid, vitamin B2, or vitamin B3 may also be a composition constituted as a single active ingredient for a direct effect of removing or preventing a hangover and improving liver function.

As used herein, the term "hangover" is a generic term for physical and mental discomfort that occurs after drinking, and refers to, as objective symptoms, complex clinical phenomena such as headache, muscle pain, heartburn, nausea, vomiting, drowsiness, decreased exercise capacity, hematologic changes, and hormonal changes. Although the exact cause of hangover is not yet found, it is generally known that a hangover occurs when the blood concentration of acetaldehyde, which is a metabolite generated during alcohol metabolism, is high.

As used herein, the term "removing" refers to any action that reduces or eliminates symptoms of a hangover by ingestion of a food composition for removing or ameliorating a hangover, including the composition according to the present specification.

As used herein, the term "amelioration" refers to any action that improves symptoms by ingestion of a food composition for removing or ameliorating a hangover, including the composition according to the present specification, for example, that at least reduces the severity of symptoms.

In one embodiment, it was found that, when a zebrafish model showing an anesthetic effect after acute alcohol treatment was treated with a mixture of omega-3 fatty acid, vitamin B2 (riboflavin), and vitamin B3 (nicotinamide), according to an embodiment, the movement distance of zebrafish was significantly increased, compared to when treated alone with each of omega-3 fatty acid, vitamin B2 (riboflavin), vitamin B3 (nicotinamide), and "X" and "Y," which are currently available hangover removal drinks (Table 1).

Through the above results, it was found that the mixture of omega-3 fatty acid, vitamin B2 (riboflavin), and vitamin B3 (nicotinamide), according to one embodiment, was effective in removing or ameliorating hangover symptoms.

Since the food composition of the present specification can be consumed on a daily basis, the effect of removing or ameliorating a hangover can be expected, and thus, the food composition may be very effectively used.

The food form of the present specification includes forms such as pills, powders, granules, needles, tablets, capsules, or liquids, and a food to which the composition of the present specification can be added may be, for example, various foods such as beverages, gum, tea, vitamin complexes, and health supplement foods.

As long as the composition is included as an essential ingredient that may be included in the food composition of the present specification, other ingredients are not particularly limited, and the food composition may include various medicinal herb extracts, a sitologically acceptable carrier, natural carbohydrates, or the like as additional ingredients like general foods. In addition, the sitologically acceptable carrier includes food supplement additives commonly used in the art, for example, a flavoring agent, a flavoring agent, a colorant, a filler, and a stabilizing agent.

Examples of the natural carbohydrates include: monosaccharides such as glucose and fructose; disaccharides such as maltose and sucrose; and polysaccharides such as general sugars, e.g., dextrin and cyclodextrin, and sugar alcohols, e.g., xylitol, sorbitol, and erythritol. As a flavoring agent other than the above-described flavoring agents, a natural flavoring agent (e.g., rebaudioside A, glycyrrhizin, and the like) and a synthetic flavoring agent (saccharin, aspartame, and the like) may be preferably used.

The sitologically acceptable carrier may include various nutritional supplements, flavors such as synthetic flavors and natural flavors, colorants and fillers (cheese, chocolates, and the like), pectic acid and salts thereof, alginic acid and salts thereof, organic acids, a protective colloid thickener, a pH adjuster, a stabilizer, a preservative, glycerin, alcohols, a carbonating agent used in carbonated beverages, and the like. In addition, the sitologically acceptable carrier may include flesh for the preparation of natural fruit juice, fruit juice beverages, and vegetable beverages. These ingredients may be used alone or a combination thereof may be used.

Another embodiment provides a health functional food composition for removing or ameliorating a hangover, including omega-3 fatty acid as an active ingredient.

The terms "omega-3 fatty acid," "active ingredient", "hangover," "removing," and "amelioration" are the same as described above.

The health functional food composition for removing or ameliorating a hangover may further include vitamin B2 (riboflavin) and vitamin B3 (nicotinamide). In this regard, vitamin B2 and vitamin B3 are the same as described above.

As used herein, the term "health functional food" is the same term as food for special health use (FoSHU), and refers to a food having high medicinal and medical effects, which is processed to efficiently exhibit bioregulatory functions as well as to supply nutrients. Here, the term "functional" refers to obtaining a useful effect for health purposes such as regulating nutrients or physiological action for the structure and function of the human body. The food of the present disclosure may be prepared by a method commonly used in the art, and may be prepared by adding raw materials and ingredients commonly added in the art. In addition, the food may also be prepared in any formulation without limitation, as long as the formulation is recognized as a food. The food composition of the present disclosure may be prepared as various formulations, and unlike general drugs, has the advantage of having no side effects that may occur during long-term administration of a drug since it contains food as a raw material, and has excellent portability. Thus, the food of the present disclosure can be ingested as a supplement for enhancing the effect of removing or ameliorating a hangover.

Another embodiment provides a method for removing a hangover, including administering, to a subject in need thereof, a composition including an effective amount of omega-3 fatty acid.

As used herein, the term "effective amount" may be administered orally or parenterally during clinical administration, and may be used in the form of general pharmaceutical formulations. Parenteral administration may refer to administration via an administration route other than an oral route, such as a rectal, intravenous, peritoneal, muscle, arterial, transdermal, nasal, inhalation, ocular, or subcutaneous route, and may be local administration to a lesion site,

and the like. For oral administration, the pharmaceutical composition may be formulated such that the active ingredient is coated in order to prevent the active ingredient from being decomposed in the stomach, or may be formulated to be protected from being decomposed in the stomach. When the pharmaceutical composition of the present disclosure is used as a medicine, the pharmaceutical composition may further include one or more active ingredients exhibiting the same or similar functions.

Another embodiment provides a food composition for use in removing a hangover, including omega-3 fatty acid as an active ingredient.

Another embodiment provides a food composition for improving liver function, including omega-3 fatty acid as an active ingredient.

Another embodiment provides a pharmaceutical composition for improving liver function or preventing or treating liver damage, including omega-3 fatty acid as an active ingredient.

The omega-3 fatty acid and the active ingredient are the same as described above.

The food composition for improving liver function may further include vitamin B2 (riboflavin) and vitamin B3 (nicotinamide). In this regard, vitamin B2 and vitamin B3 are the same as described above.

As used herein, the term "liver function" may refer to a function of maintaining metabolic homeostasis in the body through detoxification of the major nutrients such as the liver and synthesis, metabolism, storage and redistribution of carbohydrates, proteins, lipids, and the like.

The term "amelioration" refers to any action that ameliorates symptoms by ingestion of a food composition for improving liver function, including the composition according to an embodiment, when the liver function is impaired due to excessive drinking, for example, any action that at least reduces the severity of symptoms.

The food composition is the same as described above.

As used herein, the term "pharmaceutical composition" may refer to a molecule or compound that imparts several beneficial effects upon administration to a subject. Beneficial effects include: enabling diagnostic decisions; amelioration of a disease, symptom, disorder or condition; reducing or preventing the onset of a disease, symptom, disorder or condition; and responding to a disease, symptom, disorder or condition in general.

The pharmaceutical composition may further include, in addition to the active ingredient, at least one adjuvant selected from a pharmaceutically acceptable carrier, an excipient, a diluent, a filler, an extender, a wetting agent, a disintegrant, an emulsifier (surfactant), a lubricant, a sweetener, a flavoring agent, a suspending agent, a preservative, and the like. The adjuvant may be appropriately adjusted according to a dosage form to which the pharmaceutical composition is applied, and one or more of all adjuvants that can be commonly used in the pharmaceutical field may be selected and used. In one embodiment, as the pharmaceutically acceptable carrier, saline, sterile water, Ringer's solution, buffered saline, dextrose solution, maltodextrin solution, glycerol, ethanol, liposomes, and a mixture of one or more of these components, which are commonly used in drug formulation, may be used. If needed, other conventional additives such as antioxidants, buffers, and bacteriostats may be added. In addition, the pharmaceutical composition may be formulated as injectable formulations such as an aqueous solution, a suspension, and an emulsion, pills, capsules, granules, or tablets by additionally adding a diluent, a dispersant, a surfactant, a binder, and a lubricant. In addition, target organ-specific antibodies or other ligands may be used in combination with the carrier. Furthermore, the pharmaceutical composition may be preferably formulated according to each disease or ingredient by using an appropriate method in the art or a method disclosed in the Remington's document (Remington's Pharmaceutical Science (latest edition), Mack Publishing Company, Easton PA.)

Another embodiment provides a health functional food composition for improving liver function, including omega-3 fatty acid as an active ingredient.

The omega-3 fatty acid and the active ingredient are the same as described above.

The health functional food composition for improving liver function may further include vitamin B2 (riboflavin) and vitamin B3 (nicotinamide). In this regard, vitamin B2 and vitamin B3 are the same as described above.

Liver function, improvement or amelioration, the health functional food composition are the same as described above.

### BRIEF DESCRIPTION OF THE DRAWINGS

The above and other aspects, features, and advantages of certain embodiments of the disclosure will be more apparent from the following description taken in conjunction with the accompanying drawings, in which:

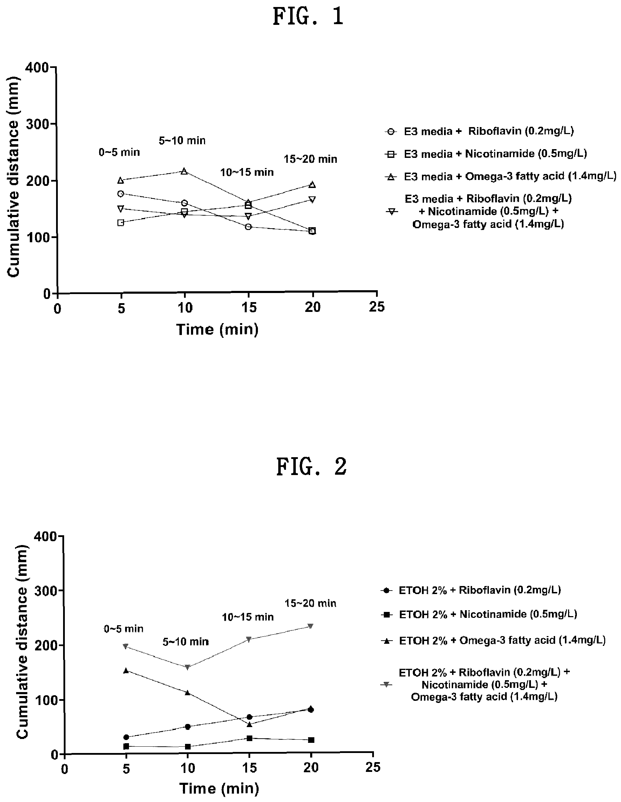

FIG. 1 is a graph showing movement tendencies over time, after a normal Zebrafish control was treated with each of omega-3 fatty acid, riboflavin (vitamin B2), nicotinamide (vitamin B3), and a mixture thereof, according to an embodiment;
FIG. 2 is a graph showing movement tendencies over time after zebrafish model experimental groups showing an anesthetic effect by acute alcohol treatment were treated with each of omega-3 fatty acid, riboflavin (vitamin B2), nicotinamide (vitamin B3), and a mixture of omega-3 fatty acid, riboflavin (vitamin B2), and nicotinamide (vitamin B3), according to an embodiment;

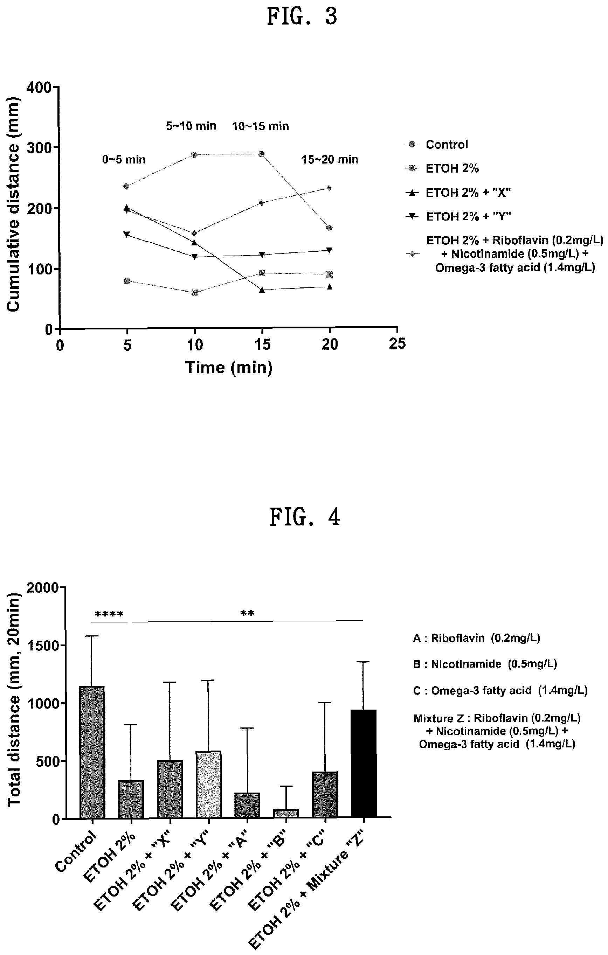

FIG. 3 is a graph showing movement tendencies over time after zebrafish model experimental groups showing an anesthetic effect by acute alcohol treatment were treated with a mixture of omega-3 fatty acid, riboflavin (vitamin B2), and nicotinamide (vitamin B3), according to an embodiment, and each of currently available hangover removal drinks "X" and "Y" from other companies (X and Y: hangover removal drinks from other companies); and
FIG. 4 is a graph showing the results of measuring movement distances after zebrafish model experimental groups showing an anesthetic effect by acute alcohol treatment were treated with each of currently available hangover removal drinks, riboflavin (vitamin B2), nicotinamide (vitamin B3), omega-3 fatty acid, and a mixture of riboflavin (vitamin B2), nicotinamide (vitamin B3), and omega-3 fatty acid, according to an embodiment (Control: Normal zebrafish control, X and Y: hangover removal drinks from other companies, A: vitamin B2, B: vitamin B3, C: Omega-3 fatty acid, Z: mixture of vitamin B2, vitamin B3, and omega-3 fatty acid).

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

Hereinafter, the present disclosure will be described in further detail with reference to the following examples. However, these examples are for illustrative purposes of one or more embodiments and are not intended to limit the scope of the present disclosure.

### Example 1. Validation of Movement Tendency of Zebrafish Model with Acute Alcohol Treatment

To compare the movement tendency of a normal zebrafish control with those of zebrafish model experimental groups showing an anesthetic effect by acute alcohol treatment for omega-3 fatty acid, riboflavin (vitamin B2), nicotinamide (vitamin B3), and a mixture thereof, the movements of the normal control and the experimental groups were observed for 20 minutes after treatment with the substances.

First, each of omega-3 fatty acid (1.4 mg/L), riboflavin (vitamin B2) (0.2 mg/L), and nicotinamide (vitamin B3) (0.5 mg/L) was dissolved in E3 media to prepare a pre-treatment solution. A mixture of omega-3 fatty acid (1.4 mg/L), riboflavin (vitamin B2) (0.2 mg/L), and nicotinamide (vitamin B3) (0.5 mg/L) was dissolved in E3 media to prepare a pre-treatment solution. Each of omega-3 fatty acid (1.4 mg/L), riboflavin (vitamin B2) (0.2 mg/L), and nicotinamide (vitamin B3) (0.5 mg/L) was dissolved in ETOH 2% (in E3 media) to prepare a post-treatment solution. A mixture of omega-3 fatty acid (1.4 mg/L), riboflavin (vitamin B2) (0.2 mg/L), and nicotinamide (vitamin B3) (0.5 mg/L) was dissolved in ETOH 2% (in E3 media) to prepare a post-treatment solution. Zebrafish larvae at 7 days post-fertilization (dpf) were exposed in water to 200 µl/well of the pre-treatment solution for each group (n=20/group) in a 96-well plate for 1 hour. 1 zebrafish larva was in each well. After 1 hour of pre-treatment, the pre-treatment solution was replaced with 200 µl/well of the post-treatment solution for each group, followed by exposure in water. Subsequently, the behaviors of the zebrafish larvae were recorded for 20 minutes. After recording, the cumulative distances for each section (0-5 min, 5-10 min, 10-15 min, and 15-20 min) of the normal control and the experimental groups were analyzed, and the results thereof are shown in FIGS. 1 and 2.

FIG. 1 is a graph showing movement tendencies over time, after a normal Zebrafish control was treated with each of omega-3 fatty acid, riboflavin (vitamin B2), nicotinamide (vitamin B3), and a mixture thereof, according to an embodiment.

FIG. 2 is a graph showing movement tendencies over time after zebrafish model experimental groups showing an anesthetic effect by acute alcohol treatment were treated with each of omega-3 fatty acid, riboflavin (vitamin B2), nicotinamide (vitamin B3), and a mixture of omega-3 fatty acid, riboflavin (vitamin B2), and nicotinamide (vitamin B3), according to an embodiment.

As shown in FIGS. 1 and 2, it was confirmed that the movement tendency of the zebrafish experimental group treated with a mixture of omega-3 fatty acid, riboflavin (vitamin B2), and nicotinamide (vitamin B3) significantly increased when compared to the experimental group treated alone with each substance.

Next, to compare movement tendencies for a mixture of omega-3 fatty acid, riboflavin (vitamin B2), and nicotinamide (vitamin B3), and currently available hangover removal drinks "X" and "Y" from other companies, the movements of the zebrafish experimental groups were observed for 20 minutes after being treated with the substances. Particularly, each of X (a hangover removal drink from other company) and Y (a hangover removal drink from another company) was dissolved in E3 media to prepare a pre-treatment solution. A mixture of omega-3 fatty acid (1.4 mg/L), riboflavin (vitamin B2) (0.2 mg/L), and nicotinamide (vitamin B3) (0.5 mg/L) was dissolved in E3 media to prepare a pre-treatment solution. Each of X (a hangover removal drink from other company) and Y (a hangover removal drink from another company) was dissolved in ETOH 2% (in E3 media) to prepare a post-treatment solution. A mixture of omega-3 fatty acid (1.4 mg/L), riboflavin (vitamin B2) (0.2 mg/L), and nicotinamide (vitamin B3) (0.5 mg/L) was dissolved in ETOH 2% (in E3 media) to prepare a post-treatment solution. Zebrafish larvae at 7 days post-fertilization (dpf) were exposed in water to 200 µl/well of the pre-treatment solution for each group (n=20/group) in a 96-well plate for 1 hour. 1 zebrafish larva was in each well. After 1 hour of pre-treatment, the pre-treatment solution was replaced with 200 µl/well of the post-treatment solution for each group, followed by exposure in water. Subsequently, the behaviors of the zebrafish larvae were recorded for 20 minutes. After recording, the cumulative distances for each section (0-5 min, 5-10 min, 10-15 min, and 15-20 min) of the normal control and the experimental groups were analyzed, and the results thereof are shown in FIG. 3.

FIG. 3 is a graph showing movement tendencies over time after zebrafish model experimental groups showing an anesthetic effect by acute alcohol treatment were treated with a mixture of omega-3 fatty acid, riboflavin (vitamin B2), and nicotinamide (vitamin B3), according to an embodiment, and each of currently available hangover removal drinks "X" and "Y" from other companies (X and Y: hangover removal drinks from other companies).

As shown in FIG. 3, it was confirmed that the movement tendency of the zebrafish experimental group treated with a mixture of omega-3 fatty acid, riboflavin (vitamin B2), and nicotinamide (vitamin B3), according to an embodiment significantly increased when compared to the experimental group treated with each of the hangover removal drinks from other companies.

The above results suggest that the mixture of omega-3 fatty acid, riboflavin (vitamin B2), and nicotinamide (vitamin B3), according to an embodiment can be effectively used in removing or ameliorating a hangover.

### Example 2. Measurement of Movement Distance of Zebrafish Model with Acute Alcohol Treatment

To compare the movement distances of zebrafish model experimental groups showing an anesthetic effect by acute alcohol treatment, for a mixture of omega-3 fatty acid, riboflavin (vitamin B2), and nicotinamide (vitamin B3), and hangover removal drinks from other companies, the movement distances of the experimental groups were observed through zebrafish video tracking for 20 minutes after treatment with the substances.

Particularly, the behaviors of zebrafish larvae were recorded for 20 minutes, and after recording, the total distance for an experiment time of 20 minutes of each of the normal control and the experimental groups was analyzed, and the results thereof are shown in FIG. 4.

FIG. 4 is a graph showing the results of measuring movement distances after zebrafish model experimental groups showing an anesthetic effect by acute alcohol treatment were treated with each of currently available hangover removal drinks, riboflavin (vitamin B2), nicotinamide (vitamin B3), omega-3 fatty acid, and a mixture of riboflavin (vitamin B2), nicotinamide (vitamin B3), and omega-3 fatty acid, according to an embodiment (Control: Normal zebrafish control, X and Y: hangover removal drinks from other companies, A: vitamin B2, B: vitamin B3, C: omega-3 fatty acid, Z: mixture of vitamin B2, vitamin B3, and omega-3 fatty acid).

The average value of the total movement distance of the experimental group treated with each ingredient and the sum of movement distances were converted into numerical values on the basis of FIG. 4, and are shown in [Table 1] below (B2: vitamin B2, B3: vitamin B3).

**[Table 1]**

| Ingredient | Average value of total movement distance (mm) | Sum of total movement distances (mm) |
|---|---|---|
| ETOH + B2 | 223.97 | K=700.66 |
| ETOH + B3 | 77.39 | |
| ETOH + Omega-3 fatty acid | 399.3 | |
| ETOH + B2 + B3 + Omega-3 fatty acid | 931.28 | L=931.28 |
| L/K(%) | | 132.9% |

As shown in FIG. 4, it was confirmed that the movement distance of the zebrafish experimental group treated with a mixture of omega-3 fatty acid, riboflavin (vitamin B2), and nicotinamide (vitamin B3), according to an embodiment was similar to that of the normal zebrafish control, and significantly increased when compared to the experimental group treated alone with each substance and each of the hangover removal drinks from other companies.

In addition, as shown in Table 1, it was confirmed that the total distance of the experimental group treated with a mixture of omega-3 fatty acid, riboflavin (vitamin B2), and nicotinamide (vitamin B3), according to an embodiment significantly increased, i.e., about 1.32 times that of the experimental group treated alone with each substance.

The above results suggest that the mixture of omega-3 fatty acid, riboflavin (vitamin B2), and nicotinamide (vitamin B3), according to an embodiment can be effectively used in removing or ameliorating a hangover.

As is apparent from the foregoing description, a composition according to an embodiment can embody an excellent hangover removal effect, and thus, the composition can be effectively used in a food composition or health functional food composition for removing or ameliorating a hangover and improving liver function.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the spirit and scope as defined by the following claims.

## Claims

1. A food composition for use in removing or ameliorating a hangover, comprising omega-3 fatty acid as an active ingredient.

2. The food composition of claim 1, wherein the omega-3 fatty acid is selected from α-linolenic acid (ALA), eicosapentaenoic acid (EPA), and docosahexaenoic acid (DHA).

3. The food composition of claim 1, further comprising a vitamin B group.

4. The food composition of claim 3, wherein the vitamin B group comprises vitamin B2 (riboflavin) and vitamin B3 (nicotinamide).

5. The food composition of claim 1, wherein the vitamin B2, the vitamin B3, and the omega-3 fatty acid are included in a concentration ratio of 1:0.5-9:5-14.

6. The food composition of claim 5, wherein the vitamin B2, the vitamin B3, and the omega-3 fatty acid is included in a concentration ratio of 1:2.5:7.

7. The food composition of claim 1, wherein the food is in the form of any one selected from powder, a granule, a tablet, a capsule, a liquid, and a beverage.

8. The food composition of claim 1, wherein the food composition consists of the omega-3 fatty acid, vitamin B2, vitamin B3, and a sitologically acceptable carrier.

9. A health functional food composition for use in removing or ameliorating a hangover, comprising omega-3 fatty acid as an active ingredient.

10. The health functional food composition of claim 9, wherein the omega-3 fatty acid is selected from α-linolenic acid (ALA), eicosapentaenoic acid (EPA), and docosahexaenoic acid (DHA).

11. The health functional food composition of claim 9, further comprising a vitamin B group.

12. The health functional food composition of claim 11, wherein the vitamin B group comprises vitamin B2 (riboflavin) and vitamin B3 (nicotinamide).

13. The health functional food composition of claim 9, wherein the vitamin B2, the vitamin B3, and the omega-3 fatty acid are included in a concentration ratio of 1:0.5-9:5-14.

14. The health functional food composition of claim 13, wherein the vitamin B2, the vitamin B3, and the omega-3 fatty acid are included in a concentration ratio of 1:2.5:7.

15. The health functional food composition of claim 9, wherein the composition consists of the omega-3 fatty acid, vitamin B2, vitamin B3, and a sitologically acceptable carrier.
